# EUROPEAN PATENT APPLICATION

(11) **EP 2 886 660 A1**
(43) Date of publication of application: **24.06.2015**
(21) Application number: 13199049.1
(22) Date of filing: 20.12.2013
(51) Int. Cl.: C12Q 1/68

(54) **A fluorescent in situ hybridization method for identification of a microorganism**

(71) Applicant: Rigas Tehniska universitate, Riga 1658 (LV)
(72) Inventor: Mezule, Linda, 1058 Riga (LV); Dalecka, Brigita, 4122 Vecpiebalga (LV); Rubulis, Janis, 2130 Dzidrinas (LV); Juhna, Talis, 2164 Baltezers (LV)
(74) Representative: Fortuna, Jevgenijs

(57) **Abstract**

The invention relates to fluorescent *in situ* hybridization (FISH) methods for identification of microorganisms. A FISH method is proposed for identification of a microorganism with at least two identically labelled oligonucleotide probes targeting different sites of rRNA of the microorganism. The method comprising the steps of (i) contacting a sample comprising a microorganism with a set of at least two identically labelled oligonucleotides under *in situ* hybridization conditions to produce a contacted sample with specific fluorescence of the microorganism; (ii) hybridization to two or more target sites on rRNA molecules of the microorganism; (iii) fluorescent visualization of the contacted sample to detect positive hybridization of said labelled oligonucleotides; (iv) reading and processing the contacted sample to detect fluorescence level of the hybridized microorganism.

## Description

### Field of invention

The invention relates to fluorescent *in situ* hybridization (FISH) methods for identification of microorganisms, in particular to the methods of *Clostridium* spp. biomass control during biobutanol fermentation *in situ.*

### Background art

Butanol obtained from biomass is called biobutanol. Biobutanol from biomass can be produced during acetone-butanol-ethanol (ABE) fermentation process carried out by certain genus *Clostridium* species bacteria [1]. Widespread biobutanol production is still limited due to the advanced technological requirements needed for the process control. Apart from engineering issues, solvent (ABE, biobutanol) production by *Clostridium* spp. (*C. acetobutylicum, C. beijerinckii*) is closely related to the quality of the microbial culture. No solvent is produced during the spore stage of the species. Presence of other microorganisms, not related to ABE production, increases the competition for the substrate and decreases the fermentation yields. Thus, the biobutanol fermentation process must be sterile (single or mixed consortium of defined species is required).

Currently used techniques to control fermentation bacteria are: (i) nonspecific methods (e.g. optical density [2]); these methods typically do not allow to detect pollution of bacteria; (ii) Fourier transform infrared spectroscopy [3], which requires specific equipment, database information, analyse only overall chemical composition of the system; (iii) flow cytometry, conventional light microscopy, fluorescent staining [12] - good tools, easily applicable, but not specific - all will show other microbial species.

Application of 16S rRNA probes in FISH for identification of microorganisms has been known for many years [6]. It has been used as a tool in differentiation between bacterial populations [7] and identification of certain species in their natural environments [8]. During the hybridization fluorescently labelled probe (oligonucleotide) binds to complementary RNA sequence in the cell according to Watson-Crick base pairing rules. In microbial FISH analyses most often 16S rRNS is used as a target genetic material because it is highly conserved for bacteria and archaea and usually present in the cells in high numbers.

The hybridization process and successful outcome depends on multiple factors, such as biophysical properties of nucleotide chains, hybridization temperature, time, sample type etc. and involves several general steps: (i) pretreatment of raw sample (when necessary); (ii) fixation, immobilization of the sample; (iii) pretreatment of preparations (when necessary); (iv) hybridization with the respective probes; (v) washing to remove unbound excess probe; (vi) mounting, visualization and documentation of the results.

The technology and the necessity of each preparation step is well described, however, the role of FISH in environmental and food analyses is far below cultivation and polymerase chain reaction (PCR). This can be explained by the fact that at the moment no universal FISH protocol exists. Depending on the sample type, oligonucleotide probe and microorganisms under investigation, variations in hybridization conditions (time, temperature and buffers) should be applied. Moreover, the probe binding can be limited by the accessibility to the complementary rRNA [9].

Low fluorescence signal, high signal-to-noise ratio, presence of inactive cell forms, e.g., spores, are often limiting to application of FISH. Over the decade due to the increase in requirements, improvements and modifications of the traditional FISH technique have been made. The protocol has become more and more complex, e. g., involving the use of multiple probes for a single organism [10], inclusion of signal amplification treatment steps [11], combination of flow cytometry and PCR with FISH or application of modified oligos (DOPE-FISH, CARD-FISH, PNA, LNA) have been introduced. Under specified conditions all these modifications have shown the applicability.

### Disclosure of the Invention

The proposed method provides visual information on the presence of target microorganisms and allows not only the detection of target cells but also the presence of spores. According to the invention the fluorescent probes with identical fluorescent marker are used targeting the same microorganism group at different rRNA sites to increase fluorescent signal intensity. Thus a fluorescent *in situ* hybridization method for the identification of a microorganism with at least two identically labelled oligonucleotide probes targeting different sites of rRNA of the microorganism is proposed. The method comprising the following steps: (i) contacting a sample comprising a microorganism with a set of at least two identically labelled oligonucleotides under *in situ* hybridization conditions to produce a contacted sample with specific fluorescence of the microorganism; (ii) hybridization to two or more target sites on rRNA molecules of the microorganism; (iii) fluorescent visualization (by using any fluorescence detection equipment, e.g. fluorescent microscope) of the contacted sample to detect positive hybridization of said labelled oligonucleotides; (iv) reading (e.g. computer based reading and processing) of the contacted sample to detect fluorescence level of the hybridized microorganisms. The labelled oligonucleotides comprise the same single fluorescent label at either 5' or 3' oligonucleotide end. The labelled oligonucleotides hybridize to different sites of rRNA molecules specific to the species or genus or the group or cluster of the microorganism.

### Brief Description of Drawings

Fig. 1 shows relative fluorescence intensity of target and non-target cells when hybridized with a single (target and negative control) or mixed probes;
Fig. 2 represents the relative fluorescence intensity of various target and non-target cells when hybridized with 2 identically labelled probes targeting *Clostridium* spp.

The research i.a. involved the selection of appropriate oligonucleotide probes targeting *Clostridium* clusters I and II which comprise all species relevant to biobutanol production.

Microbial strains used:
Target species:
   *Clostridium beijerinckii* DSM 6422
   *Clostridium acetobutylicum* DSM 792 (ATCC®824)
   *Clostridium tetanomorphum* DSM 4474 (ATCC®49273)
Non-target species:
   *Escherichia coli* ATCC®25922
   *Pseudomonas fluorescens* ATCC®13525
   *Lactobacillus acidophilus* ATCC®4356
   *Sphingomonas paucimobilis* LMKK 624

### Protocol for fermentation sample preparation:

### Fixation

Fixation of the samples is performed according to a modified protocol described by Amann et al. [4]. 1 ml of fermentation sample is put into a centrifuge tube and vortexed for several seconds to obtain homogeneous suspension. The sample is centrifuged (2 minutes, 2000g) and 0.9 ml of the supernatant is removed. Then 0.9 ml of sterile phosphate buffered saline is added (PBS, 7 mM Na₂HPO₄, 3 mM NaH₂PO₄, 130 mM NaCl, pH 7.2), vortexed to obtain homogeneous suspension and centrifuged (2 minutes, 2000g). After the second centrifugation again 0.9 ml of the supernatant is removed and simultaneously replaced with fresh PBS. Then again centrifuged (2 minutes, 2000g) and 0.9 ml of the supernatant removed. Finally, add 0.3 ml of 4% ice-cold PFA fixative, vortex to obtain homogeneous suspension and incubate at 4°C for 2 hours. After incubation centrifuge (2 min 2000g) the sample, remove 0.3 ml of the supernatant and simultaneously replace with fresh PBS, vortex to obtain homogeneous suspension. Centrifuge another 2 times. Record the final volume. For long term sample storage add ethanol (96%) to have a final concentration of 50% and store at -18°C.

### Sample preparation and hybridization

The necessary amount of the fixed sample (0.1 - 10 µl) was taken and the cells were immobilized with vacuum filtration on a cellulose-free membrane (track-etched, 0.2 µm pore size). The membrane was air-dried and placed on a glass slide. 25 µl of probe (Table 1) and buffer (Table 2) mix was applied. The final concentration of all probes on the membrane was 6.22 nanograms. The container was covered with cover glass and inserted in a humidified chamber. Then incubated at 46°C for 3 hours.

**Table 1. Probes used in the study.**

| Probe | Probe target | Probe type | sequence | Fluorescent label | Target microorganism group |
|---|---|---|---|---|---|
| Chis 150 | 16S rRNS | oligonucleotide | 5'- TTA TGC GGT ATT AAT CTY CCT TT -3' | 5' - CY3 | *Clostridium histolyticum* group (including *Clostridium* group I and II) |
| ClostI&II | 16S rRNS | oligonucleotide | 5'- TTC TTC CTA ATC TCT ACG CA-3' | 5' - CY3 | *Clostridium* group I un II |
| nonEUB | 16S rRNS | oligonucleotide | 5'- ACT CCT ACG GGA GGC AGC -3' | 5'- CY3 | Negative control |

**Table 2. Composition of hybridization and washing buffer for hybridization of biobutanol producing Clostridium spp. (adapted from Fuchs et al.[5])**

| **Constituents** | **Hybridization buffer mix** | **Washing buffer** |
|---|---|---|
| NaCl | 0.9 M | 0.9 M |
| Tris/HCl | 0.02 M | 0.02 M |
| SDS | 0.01 % | - |

### Sample washing and counterstaining

After 3 hours the cover glass was removed and 1 ml of pre-warmed (48°C) washing buffer was poured (alternatively the whole slide containing the membrane can be immersed) which was prepared according to Table 2. The sample was incubated at 48°C for 20 minutes, in the dark. Then the sample was washed with cold (4°C) distilled water for 3 seconds. Then the sample was air-dried and counterstained with 10 µg/ml DAPI or any other non-specific fluorescent dye, having absorption/emission spectra different from CY3.

### Visualization

After counterstaining, non-fluorescent immersion oil was applied and the sample was visualized under epifluorescent microscope with filter sets suitable for CY3 and counterstain used. 20 - 120 microscope fields of view were examined on presence of cells FISH negative, counterstaining positive cells. If such are present the fermentation culture is polluted and cannot be used for ABE production.

To determine the relative fluorescence intensity (RLU) at least 300 individual cells are measured with appropriate software (e.g. Media Cybernetics, Image Pro) and expressed as average fluorescence intensity of all cells in a said sample.

### Results

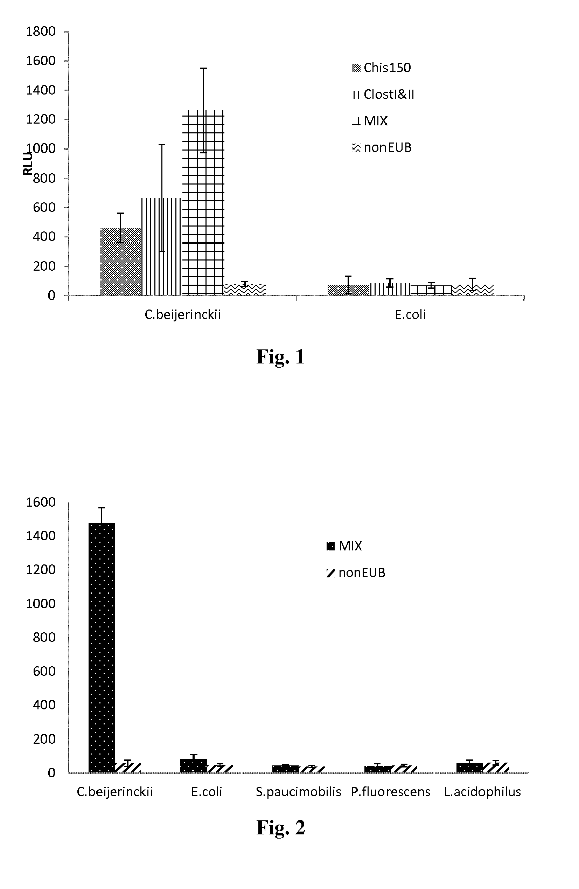

The results of measured RLU for various cultures are shown in Table 3, Fig. 1 and Table 4, Fig. 2. The data in Tables 3 and 4 represent separate sets of tests each comprising 3 full repetitions.

**Table 3. Average RLU in each sample of at least 300 individual cells measured. The data represent the average of 3 separate repetitions.**

| Probe | | Chis150 | | ClostI&II | | Mix of Chis150 and Clost I&II | | nonEUB | |
|---|---|---|---|---|---|---|---|---|---|
| Species | | CB | EC | CB | EC | CB | EC | CB | EC |
| Repetitions | 1x | 397,07 | 32,23 | 304,89 | 54,65 | 1075,14 | 47,14 | 66,16 | 39,49 |
| | 2x | 576,15 | 41,35 | 656,86 | 109,67 | 1593,22 | 74,39 | 70,96 | 62,97 |
| | 3x | 412,62 | 139,72 | 1030,91 | 96,08 | 1120,64 | 86,26 | 98,75 | 120,79 |
| | Average | 461,94 | 71,10 | 664,22 | 86,80 | 1263,00 | 69,26 | 78,62 | 74,42 |
| | STD | 99,21 | 59,60 | 363,07 | 28,66 | 286,88 | 20,06 | 17,59 | 41,84 |

| | | | | | | | | | |
|---|---|---|---|---|---|---|---|---|---|
| EC *- Escherichia coli* CB - *Clostridium beijerinckii* STD - Standard deviation | | | | | | | | | |

During the testing of the discovery no increase in the final probe concentration was performed - 50% lower final concentration of an individual probe was used in the mix. The increase in overall probe concentration (2 times) did not showed any increase in fluorescence intensity but showed only increase in background fluorescence of all samples. The choice of non-target species (Table 4) was based on the research in probe specificity and ability to bind to other microorganisms than *Clostridium.* According to Ribosomal Database Project II (http-//rd.cme.msu.edu/probematch/search.jsp) one or the other target probe showed potential rRNA complementarity to the microorganisms under test (Table 4).

**Table 4. Average RLU in each sample hybridized with probe (Chis150 and ClostI&II) mix of at least 300 individual cells measured and compared to the percentage of background fluorescence. The data represent the average of 3 separate repetitions.**

| Bacterial species | average RLU | STD | average % to background |
|---|---|---|---|
| *C.beijerinckii* | 1478,13 | 90,88 | 3,88 |
| *E.coli* | 83,60 | 26,26 | 60,44 |
| *S.paucimobilis* | 46,13 | 5,10 | 63,37 |
| *P.fluorescens* | 43,03 | 13,36 | 70,21 |
| *L.acidophilus* | 60,92 | 16,65 | 53,92 |

The inventors have discovered that in principle FISH with two or more probes targeting the same microorganism group (species, genus, etc) and possessing the same fluorescent marker, can be used in the identification of other microorganisms. The changeable parameters: probe sequences (which are specific and different for each target microbial group) and chemical composition of washing and hybridization buffers etc.

The invention can be applied not only to more distinctly distinguish the positive events (with increased fluorescence when compared to the application of a single target probe to the said microorganism) but also to monitor potential genetic shifts and decrease in rRNA content of the cells what can happen during cell stress, e.g., growth inhibiting fermentation substrate.

### Sources of information used:

1. Patakova P., Linhova M., Rychtera M., Paulova L., Melzoch K. 2013. Novel and neglected issues of acetone - butanol - ethanol (ABE) fermentation by clostridia: Clostridium metabolic diversity, tools for process mapping and continuous fermentation systems. Biotechnology Advances, 31(1), 58-67.
2. Qureshi N, Maddox IS. 2005. Reduction in butanol inhibition by perstraction: utilization of concentrated lactose/whey permeate by Clostridium acetobutylicu m to enhance butanol fermentation economics, Trans IchemE, PartC. Food Bioprod Proc;83: 43 -52.
3. Grubea M., Gavarea M., Nescerecka A., Tihomirova K., Mezule L., Juhna T. FT-IR spectroscopic analysis for studying Clostridium cell response to conversion of enzymatically hydrolyzed hay. Journal of Molecular Structure, Volume 1044, 24 July 2013, Pages 201-205.
4. Amann, R. I., B. J. Binder, R. J. Olson, S. W. Chisholm, R. Devereux, and D. A. Stahl. 1990. Combination of 16S rRNA-targeted oligonucleotide probes with flow cytometry for analyzing mixed microbial populations. Applied and Environmental Microbiology 56:1919-1925.
5. Fuchs, B. M., J. Pernthaler, and R. Amann. 2007. Single cell identification by fluorescence in situ hybridization, p. 886-896. In C. A. Reddy, T. J. Beveridge, J. A. Breznak, G. Marzluf, T. M. Schmidt, and L. R. Snyder (ed.), Methods for General and Molecular Microbiology, 3rd ed. ASM Press, Washington, D.C.
6. Amann R., Krumholz L., Stahl D.A. Fluorescent-oligonucleotideprobingofwholecellsfordeterminative, phylogenetic, andenvironmentalstudiesinmicrobiology // JournalofBacteriology. - 1990. - 172(2). - pp. 762-770.
7. Manz W., Wendt-Potthoff K., Neu T., Szewzyk U., Lawrence J. Phylogenetic composition, spatial structure, and dynamics of lotic bacterial biofilms investigated by fluorescent in situ hybridization and confocal laser scanning microscopy // Microbial Ecology. - 1999. - 37(4). - pp. 225-237.
8. Regnault B., Martin-Delautre S., Lejay-Collin M., Lefevre M., Grimont P. Oligonucleotide probe for the visualization of Escherichia coli/Escherichia fergusonii cells by in situ hybridization: specificity and potential applications // Research in Microbiology. - 2000. - 151(7). - pp. 521-533. http://www.dls.ym.edu.tw/ol_biology2/ulranet/BasePairing.html
9. Fuchs BM, Wallner G, Beisker W, Schwippl I, Ludwig W, Amann R.Flowcytometricanalysisoftheinsitu accessibilityofEscherichia coli 16S rRNAforfluorescentlylabeledoligonucleotideprobes. ApplEnvironMicrobiol. 1998 Dec;64(12):4973-82.
10. Lee S.G., Malone C., Kemp P.F., 1993. Use of multiple 16s rRNA-targeted fluorescent probes to increase signal strength and measure cellular RNA from natural planktonic bacteria. Marine ecology progress series, 101:193-201.
11. Pernthaler A., Pernthaler J., Amann R., 2002. Fluorescence in situ hybridization and catalyzed reporter deposition for the identification of marine bacteria. Appl Environ Microbiol, 68(6): 3094-3101.
12. Patakova P., Linhova M., Vykydalova P., Branska B., Rychtera M., Melzoch K. Use of fluorescent staining and flow cytometry for monitoring physiological changes in solventogenic clostridia. Anaerobe, In Press, Corrected Proof, Available online 5 November 2013.

## Claims

1. A fluorescent *in situ* hybridization method for identification of a microorganism with at least two identically labelled oligonucleotide probes targeting different sites of rRNA of the microorganism.

2. The method according to claim 1, comprising the following steps:
(i) contacting a sample comprising a microorganism with a set of at least two identically labelled oligonucleotides under *in situ* hybridization conditions to produce a contacted sample with specific fluorescence of the microorganism;
(ii) hybridization to two or more target sites on rRNA molecules of the microorganism;
(iii) fluorescent visualization of the contacted sample to detect positive hybridization of said labelled oligonucleotides;
(iv) reading and processing the contacted sample to detect fluorescence level of the hybridized microorganisms.

3. The method according to claim 1 or 2, wherein all of the labelled oligonucleotides comprise the same single fluorescent label at oligonucleotide 5' or 3' end.

4. The method according to claim 2, wherein reading is carried out by using a fluorescence detection system.

5. The method according to any preceeding claims, wherein the labelled oligonucleotides hybridize to different sites of rRNA molecules specific to the species of the microorganism.

6. The method according to any preceeding claims, wherein the labelled oligonucleotides hybridize to different sites of rRNA molecules specific to the genus of the microorganism.

7. The method according to any preceeding claims, wherein the labelled oligonucleotides hybridize to different sites of rRNA molecules specific to the group or cluster of the microorganism.

8. The method according to any preceding claims, wherein the microorganism to be identified is *Clostridium* species microorganism and the identically labelled oligonucleotide probes targeting different sites of rRNA of the microorganism are oligonucleotides having sequences 5'- TTC TTC CTA ATC TCT ACG CA -3' and 5'-TTA TGC GGT ATT AAT CTY CCT TT -3'.
